# EUROPEAN PATENT APPLICATION

(11) **EP 0 548 867 A2**
(43) Date of publication of application: **30.06.1993**
(21) Application number: 92121681.8
(22) Date of filing: 21.12.1992
(51) Int. Cl.: C12N 15/12, C12N 5/10, C07K 13/00, C12P 21/08, A61K 37/02

(54) **Soluble stem cell factor (SFC)-receptor**

(30) Priority: 23.12.1991 IL 100469; 15.10.1992 IL 103434
(71) Applicant: YEDA RESEARCH AND DEVELOPMENT CO. LTD., Rehovot 76100 (IL)
(72) Inventor: Yarden, Yosef, Rehovot 76352 (IL); Givol, David, Rehovot 76300 (IL)
(74) Representative: Grünecker, Kinkeldey, Stockmair & Schwanhäusser Anwaltssozietät

(57) **Abstract**

A soluble receptor, capable of binding a mammalian stem cell factor (SCF), comprising the extracellular domain of an SCF-receptor is disclosed. Fragments of this soluble SCF-receptor have been obtained which essentially retain the binding properties of the soluble SCF-receptor. DNA sequences encoding the soluble SCF-receptor and fragments thereof, recombinant methods for their preparation as well as antibodies directed against a soluble SCF-receptor are also disclosed.

## Description

### FIELD OF THE INVENTION

The present invention concerns a biologically active protein capable of binding a mammalian stem cell factor (SCF), which protein is a soluble SCF-receptor or an analog thereof which substantially retains the binding properties of the entire SCF-receptor for its ligand, SCF, and which may be useful for the inhibition of SCF-mediated biological effects such as those involved in certain malignant diseases or other hyperproliferative disease states, as well as for the purification of SCF.

The present invention also concerns a recombinant DNA method for preparing the above biologically active protein, a recombinant DNA molecule encoding the protein, a cell line transformed by the DNA molecule, pharmaceutical compositions containing the protein and antibodies specific for the protein.

### PRIOR ART

The following references are considered to be pertinent to the present disclosure:
**1.** Baccarini, M., Sabatini, D.M., App, A., Rapp, U.R., and Stanely, Stanley, E.R. (1990) EMBO J. 9, 3649-3657.
**2.** Basu, A., Raghunath, M., Bishagee, S., and Das, M. (1989) Mol. Cell. Biol. 9, 671-677.
**3.** Cochet, C., Kashles, O., Chumbac, E.M., Borrello, I., King, C.R., and Schlessinger, J. (1988) J. Biol. Chem. 263, 3290-3295.
**4.** Dower, S.K., Wignall, J.M., Schooley, K., McMahan, C.J., Jackson, J.L., Prickett, K.S., Lupton, S., Cosman, D., and Sims, J. (1989) J. Immunol. 142, 4314-4320.
**5.** Duan, D-S.R., Pazin, M.J., Fretto, L.J., and Williams, L.T. (1991) J. Biol. Chem. 266, 413-418.
**6.** Gunther N, Betzel C., and Weber W. (1990) J. Biol. Chem. 265, 22082-22085.
**7.** Heldin, C-H., and Westermark, B. (1990) Cell Regul. 1, 555-566.
**8.** Huang, E., Nocka, K., Beier, D.R., Chu, T-Y., Buck, J., Lahm, H-W., Wellner, D., Leder, P. and Besmer, P. (1990) Cell 63, 225-233.
**9.** Huhn, R.D., Posner, M.R., Rayter, S.I., Foulkes, J.G. and Frackelton, A.R. Jr. (1987) Proc. Natl. Acad. Sci. USA 84, 4408-4412.
**10.** Hunter, W.M. and Greenwood, F.C. (1962) Nature 194, 495-496.
**11.** Johnson, J.D., Wong, M.L., and Rutter, W.J. (1988) Proc. Natl. Acad. Sci. USA 85, 7516-7520.
**12.** Laub, O., Rall, L.B., Truett, M., Shaul, Y., Standring, D.N., Valenzuela, P., and Rutter, W.J. (1983) J. Virol 48, 271-280.
**13.** Lev, S., Givol, D. and Yarden, Y. (1991) EMBO J. 10 647-654.
**14.** Livneh E., Benveniste M., Prywes R., Felder S., Kam Z. and Schlessiner J. (1986) J. Cell Biol. 103, 327-331.
**15.** Morrison, D.K., Kaplan, D.R., Rapp, U., and Roberts, T.M. (1989) Proc. Natl. Acad. Sci. USA, 85, 8855-8859.
**16.** Morrison, D.K., Kaplan, D.R., Rhee, S.G., and Williams, L.T. (1990) Mol. Cell. Biol. 10, 2359-2366.
**17.** Paul, J.I., Tavare, J., Denton, R.M., and Steiner, D.F. (1990) J. Biol. Chem. 265, 13074-13083.
**18.** Qiu, F., Ray, P., Brown, K., Barker, P.E., Jhanwar, S., Ruddle, F.H. and Besmer, P. (1988) EMBO J. 7, 1003-1011.
**19.** Rapp U.R., Cleveland J.L., Bonner T.I. and Storm S.M. (1988) in Reddy E.P., Skalka A.M. and Curran T. (Eds) The Oncogene Handbook, Elsevier Science Pub. B.V. pp. 213-258.
**20.** Rottapel, R., Reedijk, M., Williams, D.E., Lyman, S.D. Anderson, D.M., Pawson, T., and Bernstein, A. (1991) Mol. Cell. Biol. 11, 3043-3051.
**21.** Schaefer, E.M., Siddle, K., and Ellis, L. (1990) J. Biol. Chem. 265, 13248-13253.
**22.** Scherr C.J. (1990), Am. J. Hematol. 75, 172.
**23.** Ullrich, A. and Schlessinger, J. (1990) Cell 61, 203-212.
**24.** Urlaub, G., and Chasin, L.A. (1980) Proc. Natl. Acad. Sci. USA 77, 4216-4220.
**25.** Weber, W., Gill, G.N., and Spiess, --(1984) Science 22 2294-2297.
**26.** Williams, A.F. and Barclay, A.N. (1989) Ann. Rev. Immunol. 6, 381-405.
**27.** Yarden, Y., Kuang, W-J., Yang-Feng, T., Coussens, L., Munemitsu, S., Dull, T.J., Chen, E., Schlessinger, J., Francke, U. and Ullrich, A. (1987) EMBO J. 6, 3341-3351.
**28.** Yarden, Y. and Schlessinger, J. (1987a) Biochemistry 26, 1434-1442.
**29.** Yarden, Y. and Schlessinger, J. (1987b) Biochemistry 26 1443-1451.
**30.** Yarden, Y. and Ullrich, A. (1988) Annu. Rev. Biochem. 57, 443-477.
**31.** Zsebo, M.K., Wypych, J., McNiece, I.K., Lu, H.S., Smith, K.A. Karkare, S.B., Sachdev, R.K., Yuschenkoff, V.N., Birkett, N.C., Williams, L.R., Satyagal, V.N., Tung, W., Bosselman, R.B., Mendiaz, E.A. and Langley, K.E. (1990a) Cell 63, 195-201.
**32.** Zsebo, K.M., Hsu, R-Y., Birkett, N.C. Okono, K.H., Murdock, D.C., Jacobsen, F.W., Langley, K.E., Smith, K.A., Takeishi, I., Cattanach, B.M., Galli, S.J. and Suggs, S.V. (1990b) Cell 63, 213-224.

In the following text, reference will be made to the above publications by way of statement of the respective authors and date of publication.

### BACKGROUND OF THE INVENTION AND PRIOR ART

Polypeptide growth factors are soluble mitogens that act upon their target cells after binding to specific cell surface receptors. The recently identified stem cell growth factor (SCF) is such a growth factor. This 31 kDa glycoprotein is produced by certain mesenchymal cells as a soluble or as a cell surface glycoprotein. Although little is known about the normal biological functions of SCF, the finding that its steel (Sl) locus encodes the murine SCF led researchers to believe that it plays a role in the development and migration of neural-crest derived melanocytes, germ cells and hematopoietic progenitors.

The biological effects of SCF are mediated by a 145 kDa cell surface glycoprotein, the SCF-receptor, that is encoded by the c-Kit proto-oncogene (Yarden et al., 1987; Qiu et al., 1988). The SCF-receptor shows structural similarity to other growth factor receptors and especially the receptors for the platelet-derived growth factors (PDGF's) and the macro-phage growth factor (CSF-1; reviewed by Yarden and Ulrich, 1988). These receptors share the same general structure that includes a large extracellular ligand binding region with regularly spaced cysteine residues, that define five β-strand rich immunoglobulin-like domains (Williams, 1989). A single transmembrane stretch of amino acids connects the exoplasmic portion with a cytoplasm-facing tyrosine kinase domain.

Signal transduction by growth factors is initiated by ligand-induced dimerization of the receptors (Yarden and Schlessinger, 1987a,b; Ullrich and Schlessinger, 1990). This is followed by catalytic activation of the tyrosine kinase function which in turn phosphorylates itself and several cytoplasmic proteins on tyrosine residues (Lev et al., 1991).

In analogy to the ligands of the Kit-related receptors for PDGF and CSF-1 (Heldin and Westermark 1990; Scherr, 1990), persistent stimulation of the kinase function of the SCF-receptor, as part of a chimeric molecule, resulted in phenotypic transformation (Lev et al., 1990). This suggests that SCF may be involved in malignancy or other hyperproliferative states. Interference of such SCF-mediated autocrine or paracrine loops may therefore be of very important clinical value.

To date, no attempts have been made to block SCF-mediated cellular functions in order to treat SCF-mediated disorders.

It has hitherto been contemplated to use a soluble, and hence pharmaceutically administrable, form of a cellular receptor, i.e. a truncated form of the natural membrane-bound receptor (which is usually the extracellular portion thereof carrying the ligand binding site), as a way to compete with the natural ligand and hence inhibit its biological activities. However, such an approach has met with mixed results. For example, the insulin receptor (Johnson et al., 1988; Paul et al., 1990; Schaefer et al., 1990); the interleukin-1 receptor (Dower et al., 1989); and the PDGF receptor (Duan et al., 1991) exhibit high affinity ligand binding when expressed as truncated proteins. Against this, the ligand affinity of the EGF-receptor is significantly reduced when expressed as a truncated protein representing only the extracellular domain, i.e. with the transmembrane and cytoplasmic domains removed (Weber et al., 1984; Basu et al., 1989; Günther et al., 1990).

### OBJECT OF THE INVENTION

It is the object of the present invention to provide a soluble, biologically active protein capable of binding a mammalian SCF, which may be used to compete with the natural whole SCF-receptor for binding to the natural ligand, SCF, and thereby constitute an effective antagonist for SCF-mediated biological activities. Such a protein may also be useful for the detection of SCF and for affinity based methods for the isolation and purification of SCF.

Another object of the present invention is to provide a recombinant DNA method for the preparation of the above protein, recombinant DNA molecules encoding the protein and host cells transformed by such DNA.

A further object of the present invention is to provide antibodies specific for the above protein useful for affinity based methods for purifying the above recombinant DNA-produced protein, useful for detection of natural, whole SCF-receptor, and useful for biological assays for determining the biological activities of the protein.

### GENERAL DESCRIPTION OF THE INVENTION

In accordance with the present invention it was surprisingly found that a soluble form of the SCF-receptor may be obtained which substantially retains the binding properties of the SCF receptor to its ligand, SCF.

The present invention thus provides a biologically active protein capable of binding a mammalian stem cell factor (SCF), selected from the group consisting of:
(a) soluble SCF-receptor comprising essentially the extracellular domain of an SCF-receptor; and
(b) analogs of (a) obtained by deletion, addition or replacement of one or more amino acid without substantially affecting the binding properties to SCF.

The present invention also provides a form of the above protein that is of human origin and designated Kit-X, which comprises essentially the extracellular domain of the human SCF-receptor, and analogs thereof as noted above.

The Kit-X protein according to the invention is a glycoprotein having a molecular weight of about 90 kD. The Kit-X protein of the invention retains high affinity binding to the natural ligand, SCF, and retains the ability to undergo dimerization once bound to SCF. Further, the structural integrity of the Kit-X protein compared to the whole SCF-receptor's extracellular domain is demonstrated by the immunological cross-reactivity between the two molecules whereby antibodies specific to the whole molecule cross-react with the Kit-X protein and antibodies to Kit-X protein, produced according to the present invention, cross-react with the whole receptor.

The present invention also provides, as specific embodiments of the above noted analogs, deletion mutants of the protein of the invention which comprise at least part of the SCF binding site. Specific deletion mutants are such which are capable of binding specifically to ligand-competitive antibodies raised against the entire extracellular domain of the SCF-receptor.

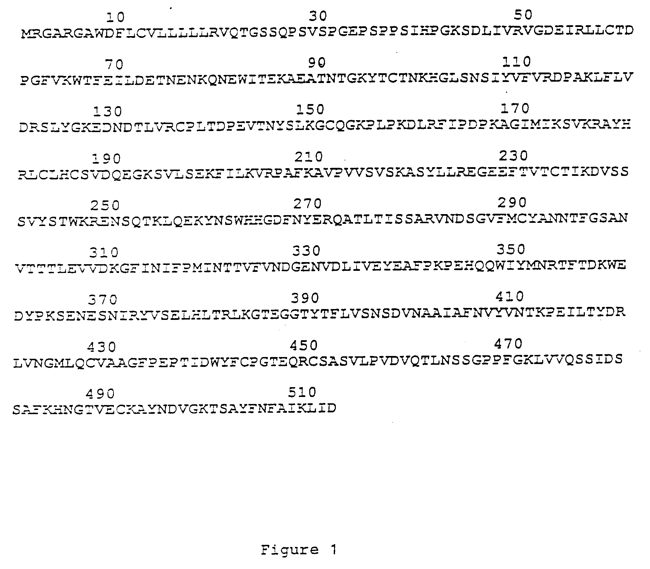

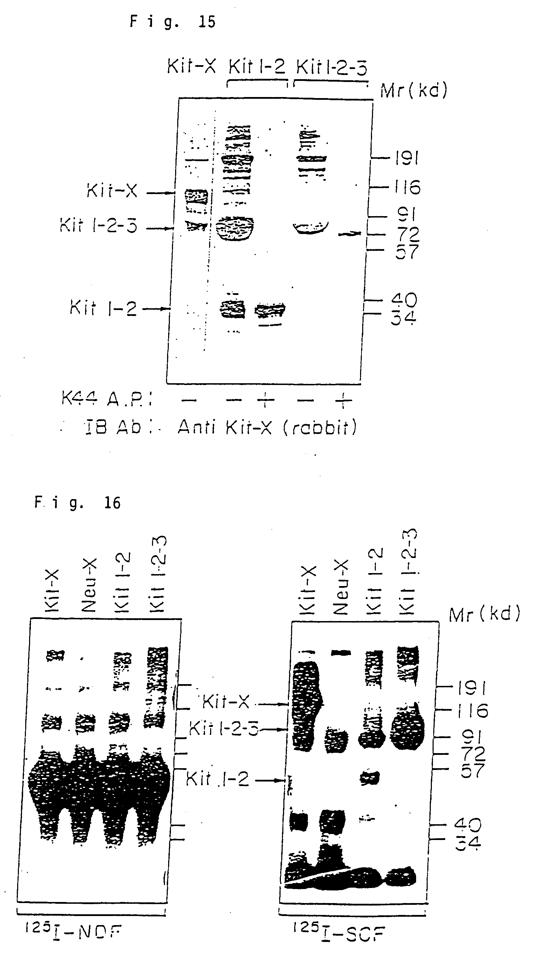

One example of the above deletion mutants is the analog of Kit-X designated herein as Kit 1-2-3 which comprises the first 3 immunoglobin-like (Ig-like) domains of the 5 Ig-like domains of the Kit-X. This analog is further characterized by having a structure schematically depicted in Figure 11 and an amino acid sequence depicted in Figure 1 from the N-terminal methionine residue (M) to about amino acid residue no. 320, an asparagine residue (N).

Another example of the above deletion mutants is the analog of Kit-X designated herein as Kit 1-2, which comprises the first 2 Ig-like domains of the 5 Ig-like domains of Kit-X. This analog is further characterized by having a structure schematically depicted in Fig. 11 and an amino acid sequence depicted in Fig. 1 from the N-terminal methionine residue (M) to about amino acid residue no. 235, an isoleucine residue (I).

The present invention also provides a recombinant DNA method for the preparation of the above biologically active soluble SCF-receptor or analogs thereof in general and of the Kit-X protein in particular, a recombinant vector and cells transfected by the vector for carrying out the method.

The recombinant DNA vector may comprise the coding sequence for the soluble SCF-receptor or analogs thereof which retains the high ligand binding affinity of the whole receptor and control sequences for its propagation and maintenance in prokaryotic host cells and/or its propagation, maintenance and expression in eukaryotic host cells.

A specific example of such a recombinant DNA vector is the eukaryotic expression vector, designated pDHFR-Kit-X comprising the Kit-X coding sequence under the control of an SV40 early promoter and a dihydrofolate reductase (DHFR) selectable marker gene under the control of a thymidine kinase promoter.

The aforesaid vectors are used according to the present invention to transfect suitable eukaryotic host cells, e.g. Chinese hamster ovary (CHO) cells, which thereby express the extracellular domain of the SCF-receptor. An example of such a cell line is that designated Kit-X-14 or EX-14 which contains the plasmid pDHFR-Kit-X and is capable of producing relatively large amounts of the Kit-X protein. A sample of this cell line was deposited at the Collection Nationale de Cultures de Microorganisms (CNCM) under Accession No. I-1085, on April 23, 1991.

The above-noted recombinant DNA method for preparing soluble SCF-receptor or analogs thereof in general and Kit-X protein in particular, comprises transfection of host cells with said expression vectors, culturing the transfected cells and recovering the soluble SCF-receptor or analogs thereof from the cultured cells and/or the culture medium.

As set forth above, the surprising and unexpected finding that the soluble SCF-receptor has retained the high ligand binding affinity and the ability to undergo dimerization when bound to ligand of the whole SCF-receptor, and the immunological cross-reactivity with the whole SCF-receptor, leads to the conclusion that this soluble SCF-receptor or analogs thereof are of potential value for the treatment of various diseases associated with SCF-mediated biological activities. In fact, as set forth in the Examples, it has been established according to the present invention, that the soluble SCF-receptor, in particular the Kit-X protein, can effectively compete with the whole SCF-receptor binding sites on binding to SCF. As a result, the Kit-X protein was capable of inhibiting the autophosphorylation by tyrosine kinase of the SCF-receptor, inhibiting the coupling to the phosphatidylinositol 3'-kinase (PI3K) and the Raf1 protein kinase, all of which events are dependent on SCF binding to the SCF-receptor. Such kinase activities constitute a major part of the signal transduction pathway of the SCF-receptor which eventually causes, inter alia, the initiation of cell proliferation.

Thus the soluble SCF-receptor of the present invention is an effective antagonist of SCF.

Accordingly, the present invention provides pharmaceutical or veterinary compositions comprising the soluble SCF-receptor e.g. the Kit-X protein, or analogs thereof and pharmaceutically acceptable carriers or excipients.

The soluble SCF-receptor or analogs thereof and pharmaceutical or veterinary compositions comprising it may be used for treating or preventing various SCF-associated diseases such as hyperproliferative or neoplastic states of myeloid, neuronal or germ cells, macrocytic anaemia, neutropenia, myeloid leukaemia, mastocytoma, glioblastoma and myelodysplasia.

The soluble SCF-receptor or analogs thereof can also be used for the detection of SCF levels in patients suspected of having SCF-associated diseases by various standard procedures known per se.

The present invention also provides the above protein (soluble SCF-receptor or analogs thereof) in a form in which it is bound to a detectable marker such as radioactive or fluorescent probes as are known per se. Further, the present invention also provides conjugates of the above protein in which the protein is bound to another biologically active molecule such as various known cytoactive drugs, cytotoxins or antibodies. These modified forms of the protein are useful as vehicles for the detection (radioactive or fluorescent probe-bound protein) or for the treatment (cytoactive drug, cytotoxin or antibody-bound protein) of disorders associated with SCF, in particular for the detection or treatment of cells which express the surface bound form of the SCF precursor molecule.

Furthermore, the soluble SCF-receptor or analogs thereof can also be used in standard affinity based isolation and purification procedures to obtain SCF.

Another aspect of the present invention is the provision of polyclonal, and monoclonal, e.g. Kit-94-IgG₁, K44, K45, K49 and K57 antibodies raised against the soluble SCF-receptor of the present invention. These antibodies are useful for detection of SCF-receptors in individuals suspected of having disorders associated with the receptor. Likewise, the antibodies are useful for standard affinity based isolation and purification procedures for SCF-receptor or for soluble SCF-receptor or analogs thereof of the invention.

### DETAILED DESCRIPTION OF THE INVENTION

### BRIEF DESCRIPTION OF THE FIGURES

- **Fig. 1**: is a representation of the nucleic acid sequence and the amino acid sequence of the truncated secreted SCF-receptor according to Example 1.
- **Fig. 2**: is a representation of the construction of the pDHFR-Kit-X vector according to Example 1.
- **Fig. 3**: is a representation of the autoradiogram (3A) and the coomassie blue and silver staining lane 1, and autoradiogram, lane 2, (3B) of the electrophoresis results according to Examples 1 and 2.
- **Fig. 4**: is a representation of the autoradiogram of the immunoprecipitation results according to Example 3.
- **Fig. 5**: is a representation of the binding of ¹²⁵I-SCF to immobilized Kit-X protein according to Example 5a (upper panel: bound vs. free SCF; lower panel: Scatchard plot).
- **Fig. 6**: is a representation of the results of the competition of Kit-X protein with ¹²⁵I-SCF for binding to T18 cells according to Example 5b (6A: competition in the presence of unlabeled SCF; 6B: competition in presence of lectin-purified Kit-X).
- **Fig. 7**: is a representation of the ligand-induced dimerization of Kit-X according to Example 6.
- **Fig. 8**: is a representation of the inhibition of tyrosine phosphorylation of SCF-receptor by Kit-X according to Example 7.
- **Fig. 9**: is a representation of the inhibition of SCF-induced PI3K activity by Kit-X according to Example 8.
- **Fig. 10**: is a representation of the inhibition of SCF-stimulated modification of Raf1 by Kit-X according to Example 9.
- **Fig. 11**: is a schematic representation of the molecular structure of the Kit-X protein, Kit 1-2-3 and Kit 1-2analogs of the invention as described in Example 10.
- **Fig. 12**: is a representation of the immunoprecipitation of Kit-X, Kit 1-2-3 and Kit 1-2 by rabbit anti-Kit-X antibodies, as described in Example 10.
- **Fig. 13**: A and B are representations of the Western blotting analysis of Kit 1-2-3 and Kit 1-2 using specific monoclonal antibodies and polyclonal antibodies, as described in Example 11.
- **Fig. 14**: is a representation of the capability of Kit-X and Kit 1-2-3 to bind to radiolabelled SCF, as described in Example 12.
- **Fig. 15**: is a representation of the Western blot analysis of Kit-X, Kit 1-2-3 and Kit 1-2, using a specific monoclonal antibody and specific polyclonal antibodies, as described in Example13.
- **Fig. 16**: is a representation of the cross-linking of the Kit-X, Kit 1-2-3 and Kit 1-2 to radiolabelled SCF, as described in Example 13.
- **Fig. 17**: is a representation of the specific interaction of Kit-X and Kit 1-2 with SCF, as described in Example 13.
- **Fig. 18**: is a representation of the specific interaction of Kit-X and Kit 1-2-3 with SCF, as described in Example 13. The invention will now be described in more detail in the following non-limiting examples and their accompanying Figures.

### EXAMPLE 1

### Construction and expression of a recombinant exoplasmic domain of the SCF-receptor:-Construction of the pDHFR-Kit-X expression vector, transfection and selection of an overexpressing CHO cell subline.

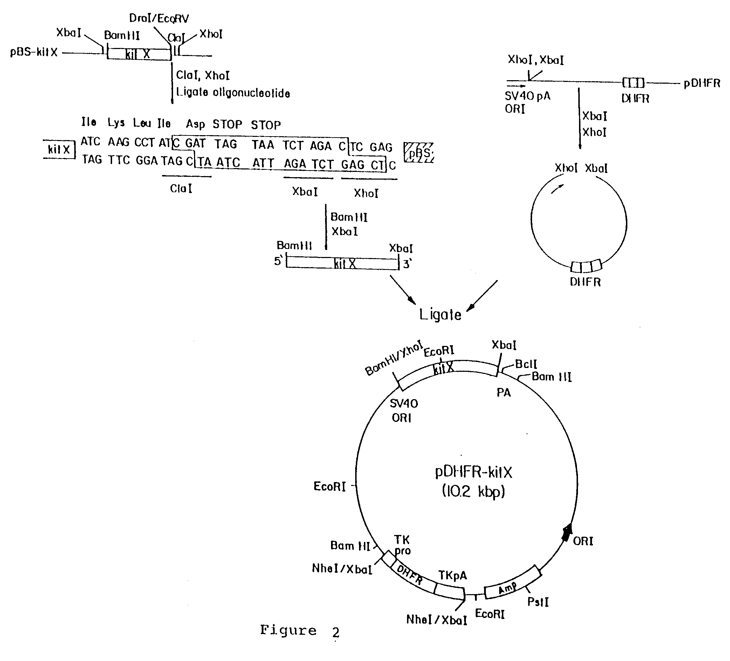

To generate a functional secreted SCF-receptor a portion of the human Kit cDNA was used (Yarden et al., 1987), that encodes a 507 amino acid protein (Fig. 1), including the signal peptide and essentially the whole extracellular domain except the 13 amino acids preceding the transmembrane region. All the cysteine residues were included in this portion in order to retain the configuration of the immunoglobulin-like domains (Barclay and Williams, 1989). Five additional amino acids from the pBS polylinker were added to the carboxy terminus by insertion of the corresponding oligonucleotides that encoded also translation stop codons and an XbaI site (Fig. 2).

An expression vector that directs the synthesis and secretion of the ectodomain of Kit (Kit-X) was constructed as follows (as shown schematically in Fig. 2): A 1560 bp BamHI-DraI fragment of the human c-Kit cDNA (Yarden et al., 1987), the amino acid sequence encoded thereby being set forth in Fig. 1 which corresponds to the extracellular portion of the SCF-receptor, was subcloned into a Bluescript (Stratagen) plasmid that was pre-cut with BamHI and EcoRV restriction enzymes. The resulting pBS-Kit-X was then cleaved open with ClaI and XhoI, and ligated into a pre-annealed pair of synthetic oligonucleotides. The nucleotide sequence of the synthetic 17 and 19 base adaptors are given in Fig. 2 together with their translation outcome. This adds 5 amino acids and two in-frame stop codons to the carboxy-terminus of the ectodomain. After ligation and transformation of bacteria the plasmid was cut with BamHI and XbaI to yield a 1.5 Kb DNA fragment that was inserted into a mammalian expression vector having an SV40 promoter and a dihidrofolate reductase (DHFR) gene as a selectable marker (pSV-DHFR). This pSV-DHFR vector represents a modification according to the present invention of the previously described pLSV vector (Laub et al., 1983; Livneh et al., 1986). This modified plasmid was cut downstream of the SV40 promoter at XhoI and Xba1 sites, and partially filled in to accommodate the BamHI terminus of the insert. The final configuration of the pDHFR-Kit-X expression vector is shown in Fig. 2. It controls transcription of the truncated Kit (Kit-X) by the SV40 early promoter, and independently directs expression of the DHFR gene by the thymidine kinase promoter.

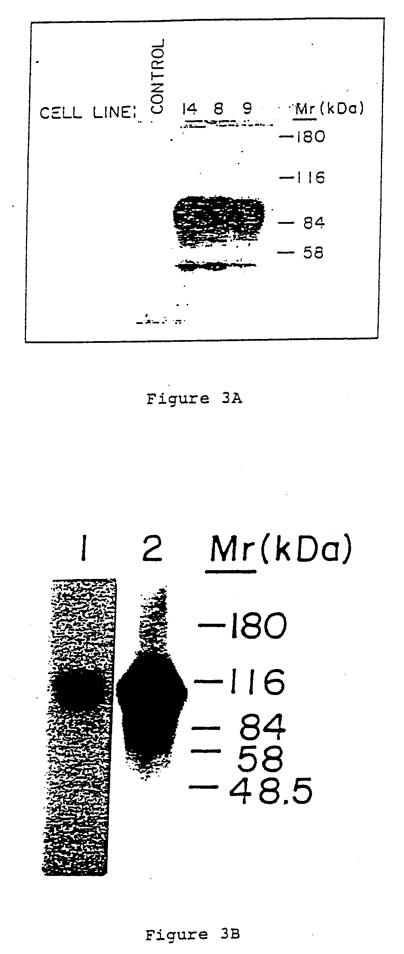

The pDHFR-Kit-X DNA was transfected into dhfr-deficient CHO mutant cells (Urlaub and Chasin, 1980) by the Ca-phosphate precipitation method and stably expressing cells were selected by their resistance to methotrexate added to nucleoside-free medium supplemented with 10% dialyzed calf serum. The resulting colonies were screened for secretion of Kit-X by metabolic labeling with ³⁵S-methionine. For metabolic labeling, colonies grown in 24-well dishes were labeled with 50µCi ³⁵S-methionine (Amersham) in 1 ml medium supplemented with dialyzed calf serum. Following 12 hr labeling the medium was harvested, cleared of cell debris and subjected to immunoprecipitation, using standard techniques, with 5 µl of an antiserum obtained from mice injected with murine fibroblasts that overexpress human c-Kit. Immunocomplexes were adsorbed to protein A on agarose beads (BioRad, Richmond, CA) and washed extensively before separation by SDS-gel electrophoresis (Fig. 3A). Positive clones underwent gene amplicification by sequential treatment with increasing concentrations of methotrexate up to 1 µM. The highest secretor clone, denoted Kit-X-14, was selected for mass-production of the protein (Fig. 3B). This analysis revealed the existence of a protein having an apparent molecular weight of about 90 kDa in the medium of transfected cells, that was absent from the medium of control untransfected cells (Fig. 3A). The apparent molecular weight of the secreted protein was much higher than the predicted molecular mass (56 kDa), and its appearance in SDS-PAGE was relatively diffuse. Both observations may be attributed to sugar residues, which contribute exactly the same mass to the full-length receptor (Yarden et al., 1987). Indeed the Kit-X protein was recognized by the lectin, wheat germ agglutinin (WGA), and could be specifically eluted from the lectin column with the corresponding sugar as set forth in Example 2 below. A cell clone designated Kit-X-14, that resisted 1 µM of methotrexate, and secreted about 4 µgr Kit-X per milliliter in 24 h, was selected for mass production of the recombinant protein.

A sample of the Kit-X-14 cell line, also designated EX-14, containing the pDHFR Kit-X vector was deposited under Accession No. I-1085 at the Collection Nationale de Cultures de Microorganisms (CNCM), Institut Pasteur, Paris, France, on April 23, 1991.

### EXAMPLE 2

### Purification of Kit-X from conditioned media -

Kit-X-14 cells (CNCM-I-1085) as described in Example 1 were grown to near confluence in 15-cm dishes. The monolayers were then washed twice with phosphate buffered saline and serum-free medium was added. Following 8-12 hrs at 37°C the medium was discarded and replaced with a minimal volume of fresh medium. The cells were left at 37°C for 2-4 days before harvest. The collected conditioned media, in batches of up to 500 ml (usually batches of 200-300 ml conditioned medium were collected), was cleared by centrifugation and concentrated 10-fold by using an Amicon ultrafiltration cell equipped with a membrane filter having molecular weight cutoff of 30,000. The resulting concentrate was subjected to affinity chromatography to purify the Kit-X protein. This affinity chromatography was carried out by mixing the above concentrate with 2-4 ml agarose beads containing wheat-germ agglutinin (WGA) (2 mg/ml packed beads). After 2 hrs of incubation, with shaking at 4°C, the beads were packed in a column that was washed extensively with Tris-HCl buffer (50 mM, pH 7.5) containing 0.5 M NaCl. Elution of bound glycoproteins was achieved with 0.3 M N-acetylglucoseamine (in 50 mM Tris-HCl, pH 7.5). The eluate was dialyzed against PBS and analyzed by gel electrophoresis to determine purity. The N-acetylglucosamine eluted fraction contained an 80-90% pure Kit-X as determined by Coomassie-Blue staining and radiolabeling (Example 4) with ¹²⁵I and lactoperoxidase (Fig. 3B).

### EXAMPLE 3

### Generation of antisera to Kit-X -

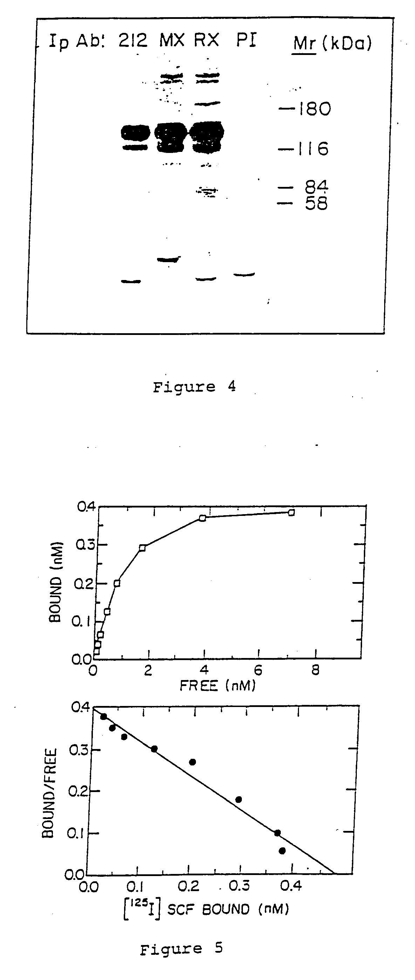

Lectin purified preparations of Kit-X (Example 2) (100 µgr each) were used for subcutaneous injection of rabbits and mice. The first injection included complete Freund's adjuvant, and the following booster injections (at 2 week intervals) contained incomplete Freund's adjuvant. Animals were bled 10 days after the third booster and the sera tested by an immunoprecipitation assay in which the antibodies were tested for recognition of the native membrane form of the SCF-receptor. As shown in Fig. 4 both the mouse (MX) and the rabbit (RX) antisera specifically immunoprecipitated the human SCF-receptor from ³⁵S-methionine labeled cells which overexpress it. The spleen of an immunized mouse was later used to generate a hybridoma which secretes a monoclonal anti-Kit-X antibody, designated Kit-94 mouse monoclonal antibody (Kit-94-IgG₁), or Kit-94 antibody (Examples 5-8 below). Other antibodies prepared from immunized rabbits (rabbit antiserum) were designated Ab212, and are specific for the carboxy terminal peptide of the Kit/SCF-receptor and were used as a positive control. These results prove the structural integrity of the purified Kit-X (Example 2) and demonstrate the usefulness of the recombinant protein for immunological purposes.

Additional monoclonal antibodies, specific for Kit-X and the ligand binding site thereof have also been prepared in the same manner, and out of these, those designated K44, K45, K49 and K57 are described in Example 11 below.

### EXAMPLE 4

### Radiolabeling of Kit-X and SCF -

Lectin-purified Kit-X (Example 2; 10 µgr) was labeled by the lactoperoxidase method as follows: 70 µl of Kit-X in PBS were mixed with 40 µl Na-acetate buffer (0.5 M, pH 5.6) and 1 mCi Na ¹²⁵I (Amersham). Lactoperoxidase (2.5 µgr/ml, 10 µl) was added and the reaction started by adding 10 µl of dilute H₂O₂ solution (0.002% in water). After 5 min at 22°C the reaction was stopped by the addition of 0.5 ml tyrosine (2 mg/ml). Radiolabeled Kit-X was separated from free iodine by gel filtration on a Sephadex G-15 column (0.8x7 cm), that was pre-saturated with gelatine (0.2% in PBS).

Recombinant human SCF made in bacteria (Zsebo et al., 1990; supplied by Amgen Corp.) was labeled by the chloramine T method (Hunter and Greenwood, 1962) and separated from free iodine by gel filtration as described above for Kit-X. The specific activity of the pooled fractions, 1-2.10⁵ cpm/ng, was calculated on the basis of a precipitation assay with trichloroacetic acid.

### EXAMPLE 5

### ¹²^{⁵}I-SCF binding to Kit-X -

### a) Direct binding to Kit-X -

Direct binding of ¹²⁵I-SCF to soluble Kit-X protein was measured by immobilization of the Kit-X as follows: Goat antibodies to mouse immunoglobulin G (IgG) were used to coat 96-well plates (50 µl/well) at a concentration of 25 µgr of the affinity-purified antibody (from Jackson, Ann Arbor, ME) per ml of PBS. Following 12 hr of incubation with the antibodies at 4°C, the goat-anti-mouse IgG antibody solution was replaced with a 20 µgr/ml solution of Kit 94 mouse monoclonal antibody to human Kit/SCF receptor (Kit-94-IgG₁, Example 3). In this way 2 layers of antibodies are formed, the first being the above goat antibodies which are overlayed by the Kit-94 monoclonal antibodies. The monoclonal antibody was removed after 3 hr at 22°C and non-specific adsorption to the wells was blocked by 2 hr incubation with PBS containing 1% BSA. Following washing the lectin-purified Kit-X (Example 2; 100 ng/well, in PBS plus 1% BSA) was added for 16 hr at 4° to bind the Kit-X to the above monoclonal antibodies and so immobilize the Kit-X protein. The plates were washed three times with PBS plus 1% BSA, and then different concentrations of ¹²⁵I-SCF (Example 4) were added. After incubation for 2 hr at 22°C the wells were washed 3 times with PBS/1% BSA and the radioactivity of individual wells was determined by the [-counter. Non-specific binding was determined by using wells that did not receive either Kit-X or the Kit-94-IgG₁ monoclonal antibody. Both resulted in less than 15% of the total binding signal. Specific and saturable binding of SCF was observed after subtraction of the non-specifically bound ligand. The results of the immobilized Kit-X assay as shown by the Scatchard analysis of the binding data (Fig. 5) revealed the presence of a single population of binding sites with a dissociation constant of 0.7 nM. Thus, the soluble form (ectodomain) of the SCF-receptor (Kit-X protein) fully retained the high-affinity binding of the intact full-length receptor for SCF (Kd of 1 nM, Huang et al., 1990).

### b) Inhibition of ¹²^{⁵}I-SCF binding to intact cells -

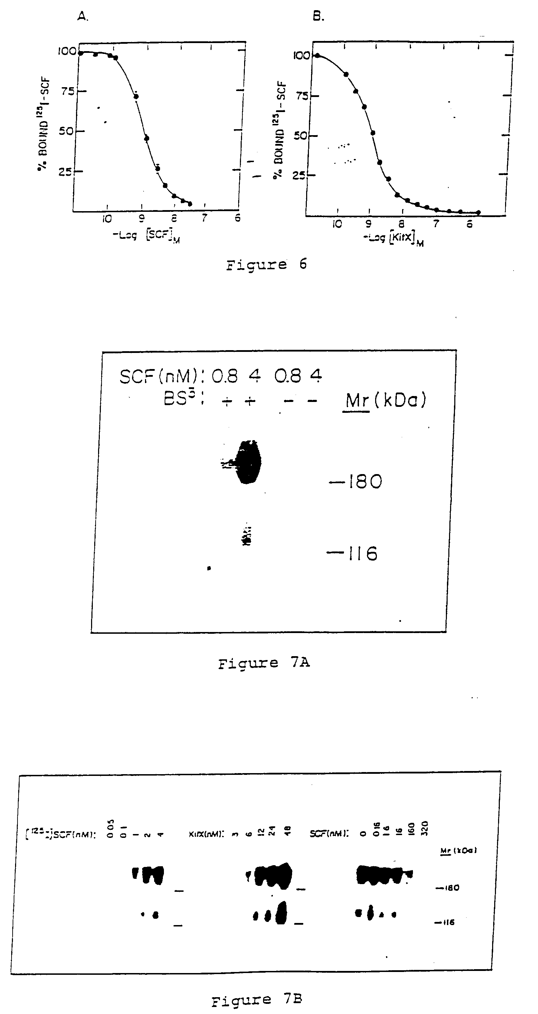

T18 cells (a CHO cell line) were established by transfection of dhfr-deficient CHO cells with the full-length cDNA human c-Kit according to the protocol described above (Example 1) for Kit-X expression. These T18 cells overexpress the entire human SCF-receptor as a result of the aforesaid transfection. For competitive binding assays with Kit-X confluent monolayers of the T18 cells in 24-well plates were used. The monolayers were washed once with binding buffer (RPMI medium containing 25 mM Hepes buffer, pH 7.5 and 0.1% BSA) and then incubated with 0.25 ml binding buffer containing a constant amount of ¹²⁵I-SCF (Example 4) and various concentrations of lectin purified Kit-X (Example 2; 0.02 nM-1.5 µM Kit-X or unlabeled SCF for comparison, as noted below). After 90 min at 22°C the cells were washed four times with binding buffer and their radioactivity determined by solubilization and harvest in 0.2N NaOH and 0.1% SDS. As depicted in Fig. 6B the soluble ectodomain (Kit-X protein) inhibited SCF binding in a concentration dependent manner. For comparison, the same experiment was repeated with increasing concentrations of unlabeled SCF (Fig. 6A). In both experiments 50% inhibition of the binding of SCF was obtained with approximately 10⁻⁹M of the competitor. Thus, the soluble Kit-X protein is capable of stoichiometric inhibition of SCF binding to the native cell surface receptor.

### EXAMPLE 6

### Covalent crosslinking of ¹²⁵I-SCF -

WGA-purified Kit-X (Example 2) was incubated with ¹²⁵I-SCF (Example 4) at various concentrations in PBS. After 2 hr at 4°C the reaction mixtures were transferred to 22°C and the proteins were crosslinked by adding bis (sulfosuccinimidyl) suberate (BS₃, Pierce). 30 min later the reaction was terminated with 150 mM glycine-HCl buffer (pH 7.5). Sepharose-protein A coupled to Kit 94 antibody (Example 3) was added 5 min later, and the Kit-X was immunoprecipitated and resolved by electrophoresis on 5.5% acrylamide gels. The results of the electrophoresis show primarily a radioactive band of an apparent molecular weight of about 220 kDa (Fig. 7A). A faint band of a molecular weight of about 110-120 kDa was also resolved. The appearance of both labeled bands was strictly dependent on the presence of the crosslinking reagent (Fig. 7A). In addition, crosslinking in the absence of SCF followed by Western blotting showed only the 110 kDa form (data not shown). Based on the analogy between the SCF-receptor and the receptors for PDGF (Heldin and Westermark, 1990; Duan et al., 1991) and EGF (Cochet et al., 1988) it can be assumed that the lower molecular weight band represents the monomeric Kit-X crosslinked to radiolabeled SCF (18 kDa), whereas the higher species is due to a dimer of the soluble ectodomain that binds one or two molecules of the ligand. The specificity and saturability of the dimerization reaction was examined by performing the chemical crosslinking reaction in the presence of increasing concentrations of either ¹²⁵I-SCF (Fig. 7B, left panel), unlabeled Kit-X (Fig. 7B, middle panel), or unlabeled SCF as a competitor (Fig. 7B, right panel). Besides demonstrating the specificity to SCF, this analysis showed that the quantitative relationships between the monomeric and the dimeric forms of the truncated receptor were not significantly affected when the concentrations of the ligand or the soluble receptor varied by two orders of magnitude; the dimeric form predominated under all conditions.

Based on these results it would appear that the exoplasmic domain of the SCF-receptor, i.e. Kit-X protein, is sufficient for dimer formation, unlike the case with the epidermal growth factor receptor (Gunther et al., 1990). A secreted ectodomain of the PDGF-receptor also undergoes ligand-induced dimerization (Duan et al., 1991). However, the monomeric form predominated in the PDGF-receptor case, unlike in the case of the Kit-X protein where the dimeric form accounted for most of the radioactively labeled protein.

### EXAMPLE 7

### In vivo tyrosine phosphorylation assay -

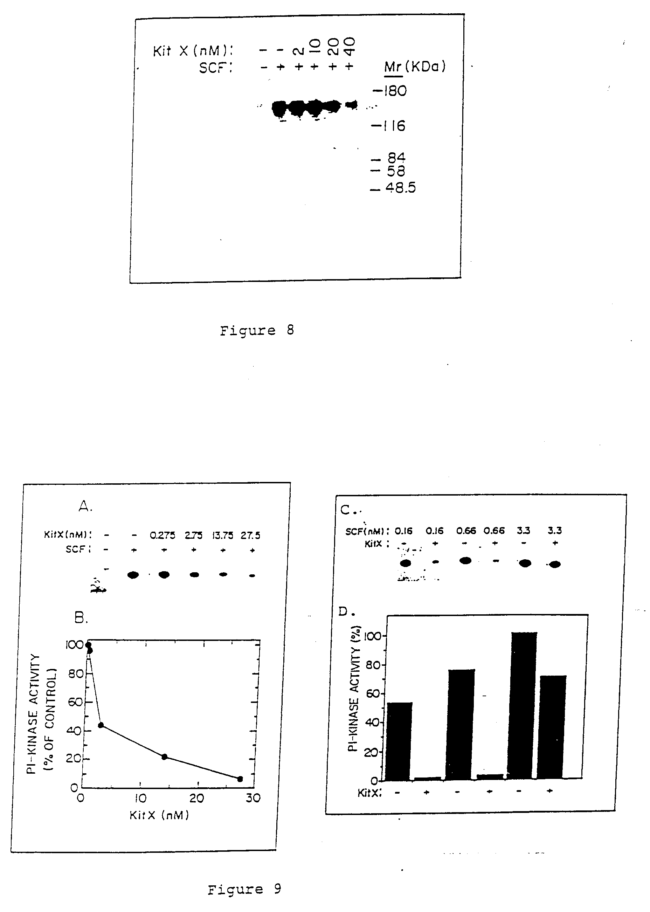

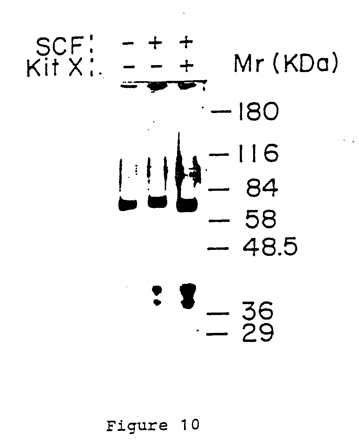

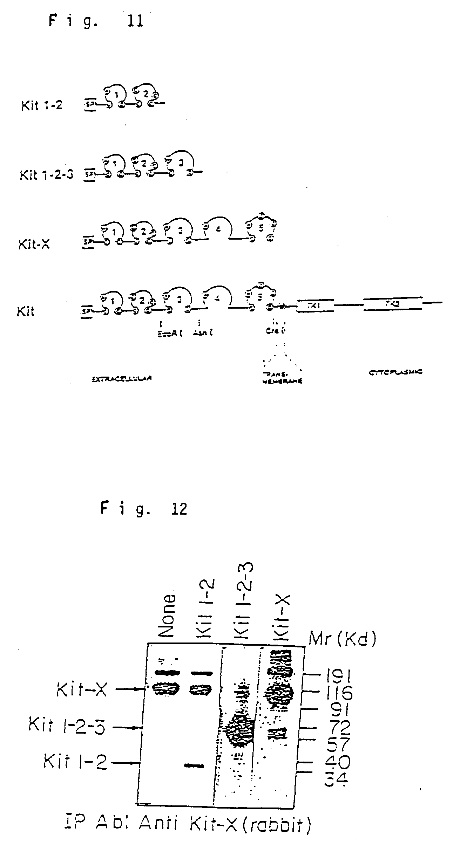

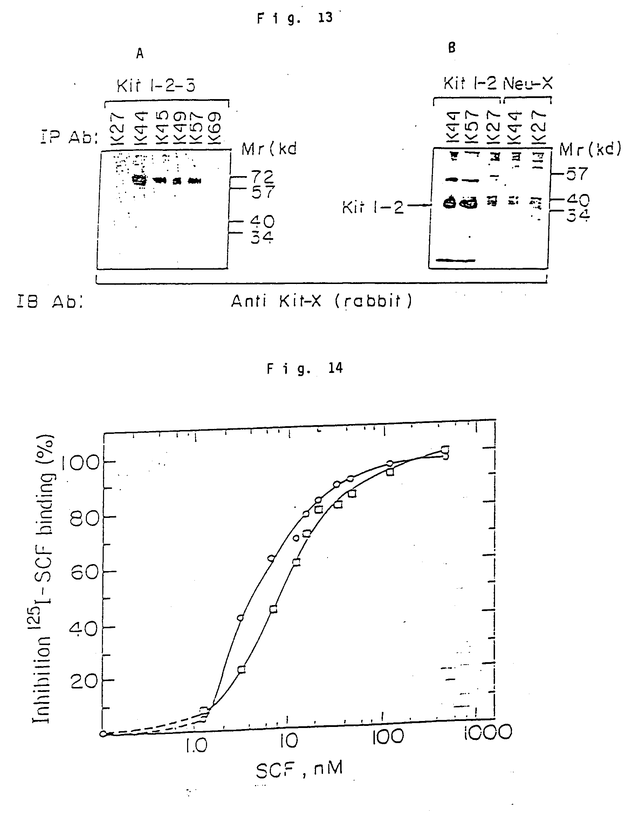

T18 cells (Example 5(b)) were grown to confluence in 6-well plates, and then labeled for 4 hours by incubation at 37°C with phosphate--free Dulbecco's modified Eagle medium supplemented with 0.1% dialyzed calf serum and 0.5 mCi ³²P-orthophosphate (9,000 Ci/mmol, Kamag, Beer Sheva). At the end of the labeling period SCF (supplied by Amgen Corp. - Example 4) was added to final concentration of 3.3 nM. Lectin-purified Kit-X (Example 2) was also added to some wells at various concentrations as indicated (Fig. 8). Stimulation by SCF lasted 15 min at 22°C and was followed by solubilization of the cells in 1 ml lysis buffer (50 mM Hepes-NaOH pH 7.5, 150 mM NaCl, 10% glycerol, 1% Triton X-100, 1.5 mM MgCl₂, 1 mM EGTA, 10 µgr/ml leupeptin, 10 µgr/ml aprotinin, 1 mM PMSF, 0.2 mM Na-orthovanadate and 100 mM Na-fluoride). Unsolubilized material was removed by centrifugation at 13,000g (15 min, 4°C), and the cleared lysate was incubated with Sepharose coupled to monoclonal antibody to phosphotyrosine (1G2, Huhn et al., 1987). Unbound proteins were removed after 45 min at 4°C by washing with HNTG (20 mM Hepes-NaOH pH 7.5, 150 mM NaCl, 0.1% Triton X-100, 10% glycerol). The adsorbed proteins were eluted by incubating the agarose beads with HNTG containing 50 mM phenyl-phosphate. Recovered protein were incubated with Sepharose-bound antibodies (from a rabbit antiserum) directed to the carboxy terminal peptide of Kit/SCF-receptor (Ab212) (Example 3). This was followed by boiling in gel sample buffer and SDS-gel electrophoresis for detection of the tyrosine-phosphorylated Kit/SCF receptor. The ability of the recombinant Kit-X protein to compete with SCF for binding to the intact surface receptor (Example 5, Fig. 6) suggested that Kit-X may antagonize the biochemical action of SCF. As receptor autophosphorylation on tyrosine residues is one of the earliest events in the signal transduction pathway of Kit, the antagonism of this function was examined as per the above method. The results of this examination are represented in Fig. 8: No tyrosine phosphorylation of the receptor took place in the absence of SCF. Incubation with the ligand induced strong labeling of the receptor band, but this was gradually decreased when SCF was given in the presence of increasing concentrations of Kit-X. It is therefore concluded that Kit-X is capable of inhibiting self-phosphorylation of the Kit kinase following stimulation wit SCF. This is very likely due to the inhibition of SCF binding to the cell bound receptors.

### EXAMPLE 8

### Assay of PI3K activity -

Confluent monolayers of T18 cells (5x10⁵ cells) (Example 5(b)) were incubated with 3 nM of human recombinant SCF (supplied by Amgen Corp.) and Kit-X (Example 2) at various concentrations for 10 min at 22°C. The cells were then solubilized in lysis buffer and the Kit/SCF-receptor immunoprecipitated with Ab-212 (anti-C-terminal peptide) (Example 3). PI3K activity in the immunoprecipitates was assayed as previously described (Lev et a₁₂l., 1991). It has been shown previously that ₁upon ligand activation of the Kit tyrosine kinase it couples to several signalling molecules including the phosphatidylinositol 3'-kinase (PI3K) and the Raf1 protein kinase (Lev et al., 1991; Rottapel et al., 1991). Therefore the capacity of the soluble Kit-X protein to antagonize these functions was examined. To measure coupling of the SCF receptor to PI3K the above-noted procedure was carried out. The results of this analysis (Fig. 9) are presented as an autoradiogram of the in vitro phosphorylated phosphatidylinositol (Fig. 9A) and by densitometric analysis of the autoradiogram (Fig. 9B). As is evident from the results low if any association of PI3K with the SCF receptor could be seen in the absence of the ligand (SCF). Once incubated with 3 nM SCF the receptor coupled to PI3K activity, but this could be inhibited almost completely upon simultaneous incubation of the cells with the Kit-X protein. The extent of inhibition by Kit-X was found to be dependent on the concentration of the ligand used for cell stimulation (Fig. 9C,D). In this analysis the above-noted procedure was carried out except that the amount of Kit-X used was 16 nM, while the amount of ligand, SCF, was varied, and as controls cells were incubated with each concentration of SCF without addition of Kit-X. The results (Fig. 9C,D) show that whereas 16 nM concentration of Kit-X inhibited, by more than 90% (Fig. 9D) the response to 0.16 nM of SCF, the same concentration of Kit-X yielded only 30% reduction of the PI3K signal elicited by 3.3 nM of SCF (Fig. 9D).

### EXAMPLE 9

### Determination of Raf1 electrophoretic mobility shift -

Confluent monolayers of T18 cells (5x10⁶ cells; Example 5(b)) were stimulated with 3.3 nM SCF at 37°C, in the presence or absence of Kit-X, as indicated in Fig. 10. The Raf1 protein was then immunoprecipitated, with an anti-peptide antiserum, from the stimulated cells, gel electrophoresed and Western blotted with antibodies to Raf1 was previously described (Lev et al., 1991). The Raf1 serine/threonine kinase undergoes phosphorylation in response to treatment of cells with a variety of mitogens (Rapp et al., 1988). Both the receptors for PDGF (Morrison et al., 1990) and CSF1 (Baccarini M. et al., 1990) induce phosphorylation and subsequent catalytic activation of the Raf1 kinase. The Kit/SCF receptor also couples to the Raf1 kinase upon ligand stimulation (Lev et al., 1991). To determine if the Kit-X protein is capable of antagonizing the effect of SCF on the Raf1 kinase the above procedure was carried out. The results (Fig. 10) show that SCF induced a reduction of the electrophoretic mobility of the Raf1 protein, indicative for its hyperphosphorylation (Morrison et al., 1989). Co-incubation of the cells with SCF and Kit-X abolished the mobility shift of Raf1 (Fig. 10), thus revealing the capability of Kit-X to antagonize also this function of SCF.

### EXAMPLE 10

### Construction and Expression of Mini-Kit-X Proteins

To construct expression vectors that direct the synthesis of portions of the extracellular domain of Kit, the human Kit-X cDNA cloned into the pBluescript (pBS) plasmid as described in Example 1 above, was used (see Fig. 1 which shows the amino acid sequence of Kit-X of 512 amino acids and which is encoded by a 1526 bp sequence).

The Kit 1-2 expression vector, containing the 5' 719 bp (240 amino acids) of human c-Kit cDNA was constructed as follows: An EcoRI - ClaI fragment was cut out from the pBS-Kit-X plasmid and the resulting ends were then filled-in with Klenow I enzyme and ligated with T4 ligase. This resulted in the juxtaposition of two in-frame stop codons 3' to the c-Kit region that encodes Ig-like domains 1 and 2. A BamHI-XbaI fragment (704 bp or 235 amino acids) was cut out from the resulting pBS and inserted into the pLSV-DHFR mammalian expression vector (Example 1, above) that was pre-cut with XhoI and XbaI downstream of the internal SV 40 early promoter.

The Kit 1-2-3 expression vector, containing 960 bp (or 320 amino acids) from the 5' end of human c-Kit was similarily constructed from pBS-Kit-X plasmid that was cleaved with BamHI and AsnI restriction endonucleases to yield a 960 bp fragment. This was ligated into a BamHI-SalI cut shuttle vector (PATH2). After transformation of bacteria the resulting plasmid was cut with BamHI and the ClaI and the gel-purified fragment was ligated into pBS-Kit-vector from which the c-Kit was removed by using BamHI and ClaI. The resulting plasmid contained the 5' 960 bp (320 amino acids) of c-Kit and an in-frame stop codon at its 3' end. A BamHI-XbaI fragment was then extracted from this plasmid and fused with a XhoI and XbaI cut pLSV-DHFR vector.

The mammalian expression vectors encoding Kit 1-2 and Kit 1-2-3, illustrated schematically in Figure 11, were then separately co-transfected with a plasmid that confers neomycin resistance into *dhfr*-negative CHO cells (Example 1, above). It should be noted that the various proteins in Figure 11 are drawn to scale, and the Ig-like domains of the various Kit proteins are indicated at the bottom of Fig. 11. Also shown are the locations of the unique restriction endonuclease sites used in the construction of the recombinant proteins. The numbers 1-5 indicate the respective Ig-like domains. The locations of all the cysteine residues of the extracellular domains are indicated by a circled C, the tryptophan residues belonging to the consensus structure of Ig-homology are shown by circled W; the designations SP indicating a signal peptide; and TK indicating tyrosine kinase. Stably expressing cells were selected by their ability to grow in gentimycin (0.8 mg/ml) containing medium. Single-cell colonies were screened by biosynthetic labeling and immunoprecipitation of the corresponding ³⁵S-methionine-labeled Kit protein (see Examples 2, 4, 5 and 6 above).

A representative immunoprecipitation analysis of the selected clones is shown in Fig. 12. Monolayers of the selected cell lines and a control untransfected cell line were incubated for 16 hrs with ³⁵S-methionine. The biosynthetically labeled Kit proteins that were secreted into the growth medium were then immunoprecipitated with a polyclonal rabbit antibody that was raised against the whole extracellular domain. As shown in Fig. 12, a 36 kilodalton protein was detectable in the medium of cells that were transfected with the Kit 1-2 plasmid, whereas a 65 kilodalton protein was secreted by Kit 1-2-3 transfected cells. These molecular weights are in agreement with the expected sizes of the recombinant glycosylatead proteins (Fig 11 and Qiu et al., 1988). In addition to the indicated P36 and p65 proteins, two other bands of 120 and 180 kilodalton appeared in the immunoprecipitates. These large molecules are attributed to non-specific interactions with the polyclonal antiserum as they were precipitated also from untransfected cells used as a control, depicted as "None", in Figure 12.

This conclusion was further supported by Western blot analyses as described in Example 11 below.

### EXAMPLE 11

### Western blotting:

Washed immunoprecipitates, as described in Example 10 above, were mixed with SDS gel sample buffer and subjected to electrophoresis on SDS-PAGE. The gel-separated proteins were electrophoretically transferred onto nitrocelluloses filters. Filters were first saturated for 1 hr at 22°C with blocking solution (10% low fat milk in 20 mM Tris-HC1 pH 7.6 and 17 mM NaC1). Antisera were then added in the same solution and incubation was carried out for 1 hr. For detection the filters were washed three times (5 min each wash) with PBS/0.05% Tween-20 and reacted for 45 min. at room temperature with horseradish peroxidase-conjugated protein A. The enzyme was removed by washing as above. The filters were reacted for 1 min with a chemiluminescence reagent (ECL, Amersham) and exposed to an autoradiography film for 1-15 min.

For the Western blot analysis, samples of each of the Kit 1-2-3, Kit 1-2 and Neu-X proteins were immuno-precipitated with the various monoclonal antibodies (K27, K44, K45, K49, K57 and K59; Fig. 13 panels A & B) and then the resulting immunocomplexes were resolved by SDS-PAGE, transferred to nitrocellulose filters and immunoblotted (IB) with the rabbit polyclonal antibody to Kit-X.

The results of the Western blotting analysis are shown in Fig. 13, panels A and B, from which it is observed that Kit 1-2-3 was recognized by four monoclonal antibodies, designated K44, K45, K49 and K57, of which K44 and K57 were shown to be ligand competitive (results not shown herein). These monoclonal antibodies were prepared by standard procedures using purified Kit-X protein (see Example 2 above) as immunogen in mice. A series of monoclonal antibodies were obtained and tested for their ability to compete with Kit-X protein for binding to ligand, i.e. to SCF, of which K44 and K57 were observed to be the most effective ligand-competitive antibodies, namely that these recognize specifically the ligand binding sites of Kit protein. In addition, these two antibodies, K44 and K57 were also capable of immunoprecipitating the shorter mutant (Kit 1-2) as shown in Fig. 13 panel B, implying ihat their epitopes are confined to IG-like domains 1 and 2 and the intervening sequences. It should also be noted that in Fig. 13, panel B, there is shown the control, designated Neu-X which is the extracellular domain of the Neu protein. As observed in Fig. 13 panel B, the antibodies K44 and K27 do not immunoprecipitate the Neu-X protein, the K27 antibody also does not immmunoprecipitate the Kit 1-2 protein, while K44 does immunoprecipitate Kit 1-2 protein. The bands observed in the lanes designated K27 for Kit 1-2 and K44 and K27 for Neu-X represent non-specific bands resulting from the interaction of the immunoprecipitation antibodies (IP) with the immunoblotted antibodies (IB), a rabbit polyclonal antibody to Kit-X (see Example 3, above).

Thus, it may be concluded that the portion of the Kit protein included in the short deletion mutant Kit 1-2 protein, and the larger deletion mutant Kit 1-2-3 contains the binding sites for the ligand-competitive antibodies K44 and K57 and accordingly also the ligand, SCF, binding site or at least an essential part thereof, of the Kit-X protein.

### EXAMPLE 12

### Direct binding of radiolabeled SCF to soluble Kit proteins:

In order to analyse the binding of radiolabeled recombinant SCF (Zsebo et al., 1990b) to Kit proteins, each protein was first immunoprecipitated by using specific rabbit polyclonal antibodies (see Example 3, above). Binding of ¹²⁵I-SCF to the immobilized proteins was performed in the presence of increasing concentrations of unlabeled SCF and was followed by extensive washing of unbound ligand (according to the procedure described above in Example 5(a). As shown in Fig. 14 both Kit-X (indicated by squares) and Kit 1-2-3 (indicated by circles) displayed a slightly different displacement curve with specific and saturable binding having apparent ligand affinities of 9 nM and 4.5nM, respectively (see also the results described with respect to Kit-X in Example 5(a) above). In contrast, no specific binding was detectable when Kit 1-2 was analysed by using the ligand-displacement assay (data not shown). Replacement of the polyclonal antibodies, which were used for immunoprecipitation of the Kit proteins, with mAbs K45 and K49 (see Example 11 above), also failed to detect specific SCF binding to Kit 1-2. It was thus concluded that despite the immunological recognition of Kit 1-2 by ligand-competing mAbs (Example 11 above), structural determinants that are included in Ig-like domain 3 participate directly or indirectly in the definition of the ligand binding cleft.

### EXAMPLE 13

### Covalent crosslinking of radiolabeled SCF to soluble Kit proteins

The possibility that the Kit 1-2 protein contains only part of the SCF binding site led to the examination of the possibility that only one component of its ligand recognition - either association or dissociation- is defective. Hence it was attempted to inhibit completely ligand dissociation by covalent crosslinking of SCF-Kit complexes (see Example 6 above with respect to procedure). To this end the recombinant proteins were purified and concentrated from cell supernatants by immunoaffinity chromatography on a column of the K44 MAb (see Example 11 above with regards the monoclonal antibody K44). Western blot analysis (see Example 11 above) of the purified proteins indicated the existence of a single immunoreactive species in each preparation (Fig. 15). The isolated proteins were then incubated with ¹²⁵I-SCF in a small reaction volume, and the covalent crosslinking reagent disuccinimidylsuberate (DSS) was later added in order to stabilize the complexes. Upon separation by gel electrophoresis, without immunoprecipitation, radioactive bands in sizes that correspond to complexes of ¹²⁵I-SCF with the respective Kit protein were observed (indicated by arrowheads in Fig. 16). Thus, crosslinking of Kit 1-2-3 yielded a 95 kilodalton band whereas Kit 1-2 yielded a crosslinked product with an apparent molecular weight of 55 kilodalton. Considering the existence of SCF as a non-covalently held dimer (monomer molecular weight - 18 kilodalton), the observed molecular sizes are consistent with the possibility that one or two molecules of SCF were bound to Kit 1-2-3 (65 kilodalton) and Kit 1-2 (36 kilodalton). An 85-kilodalton protein band appeared in all the crosslinking reactions and was therefore considered to be non-specific. As a control for the covalent crosslinking experiments the entire soluble ectodomain (Kit-X) and the recombinant ectodomain of the Neu protein (Neu-X) were used. Whereas the Kit-X underwent crosslinking to ¹²⁵I-SCF and also exhibited dimer formation, the Neu-X protein (110 kilodalton) yielded two major radioactive bands (38 and 85 kilodalton), that are probably due to non-specific interaction with ¹²⁵I-SCF. In order to confirm the specificity of the crosslinked protein bands that were obtained with ¹²⁵I-SCF, the experiment was repeated with another radiolabeled polypeptide, designated NDF that is known not to interact with Kit. As expected, it appeared that none of the three soluble Kit proteins interacted specifically with ¹²⁵I-NDF (Fig. 16).

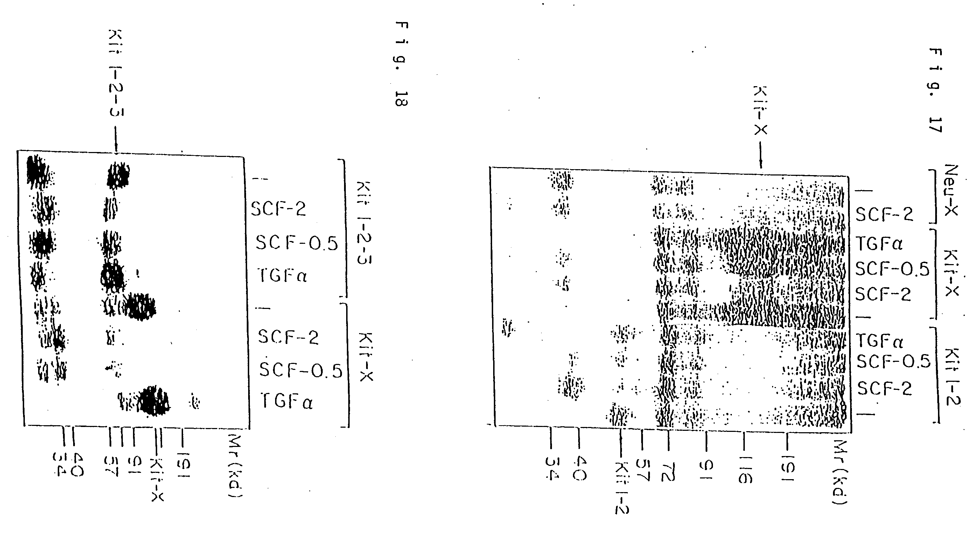

Additional experiments were performed in order to test the specificity of interaction of ¹²⁵I-SCF with the recombinant Kit proteins (Fig. 17 and Fig. 18). These analyses showed that unlabeled SCF (at 0.5 or 2nM), but not unlabeled TGFα, reduced in a concentration-dependent manner the amount of radioactivity of the protein bands that correspond to Kit 1-2-3 (Fig. 18) and Kit 1-2 (Fig. 17). Similar effects were observed when the crosslinking to Kit-X was examined (Fig. 17 and 18). However, we noted some variation in the efficiency of competition by SCF when the various Kit proteins were compared. Once again no specifically crosslinked bands were seen with the Neu-X protein (Fig. 17). It was therefore concluded that the interactions between ¹²⁵I-SCF and the recombinant proteins Kit 1-2-3 and Kit 1-2 are both ligand-specific and receptor-specific.

## Claims

1. A biologically active protein capable of binding a mammalian stem cell factor (SCF), selected from the group consisting of:
(a) soluble SCF-receptor comprising essentially the extracellular domain of an SCF-receptor; and
(b) analogs of (a) obtained by deletion, addition or replacement of one or more amino acid residues without substantially affecting the binding properties to SCF.

2. The protein according to Claim 1, selected from the group consisting of:
(a) soluble SCF-receptor designated Kit-X, comprising essentially the extracellular domain of the human SCF-receptor, and having the amino acid sequence depicted in Fig. 1; and
(b) analogs of (a) obtained by deletion, addition or replacement of one or more amino acid residues without substantially affecting the binding properties to SCF.

3. A protein according to Claim 1 or 2, selected from the group consisting of:
(a) analog of Kit-X designated Kit 1-2-3 comprising the first 3 immunoglobulin-like (Ig-like) domains of the 5 Ig-like domains of Kit-X, having a structure schematically depicted in Fig. 11 and an amino acid sequence depicted in Fig. 1 from the N-terminal methionine residue (M) to about amino acid residue no. 320; and
(b) analog of Kit-X designated Kit-1-2, comprising the first 2 Ig-like domains of the 5 Ig-like domains of Kit-X, having a structure schematically depicted in Fig. 11 and an amino acid sequence depicted in Fig. 1 from the N-terminal methionine residue (M) to about amino acid residue no. 235.

4. A protein according to any one of Claims 1-3 bound to a detectable marker, being a radioactive or fluorescent probe.

5. A conjugate comprising a protein according to any one of Claims 1-3 bound to another biologically active molecule selected from the group consisting of cytoactive drugs, cytotoxins and antibodies.

6. The Kit-X according to Claim 2, having the following characteristics:
(i) it is a glycoprotein;
(ii) it has an apparent molecular weight of about 90 kD as determined by SDS electrophoresis;
(iii) it has an affinity of binding to SCF of about Kd 1nM;
(iv) it undergoes dimerization when bound to SCF; and
(v) it has an immunological cross-reactivity with the whole SCF-receptor.

7. The Kit-X protein or analog according to any one of Claims 2, 3 and 6, encoded by a DNA sequence included in plasmid pDHFR-Kit-X contained in the Chinese hamster ovary (CHO) cell line deposited in the Collection Nationale de Cultures de Microorganisms (CNCM) under Accession No. I-1085.

8. A pharmaceutical composition comprising a protein or conjugate according to any one of Claims 1 to 7 and a pharmaceutically acceptable carrier or excipient.

9. An isolated or recombinant DNA sequence encoding the protein according to any one of Claims 1-3, 6 and 7.

10. A recombinant DNA vector comprising the DNA sequence of Claim 9 and control sequences for its propagation and maintenance in prokaryotic host cells and/or its propagation, maintenance and expression in eukaryotic host cells.

11. A vector according to Claim 10, being the plasmid pDHFR-Kit-X contained in the cell line deposited at the CNCM under Accession No. I-1085.

12. A cell line transformed by a vector according to Claim 10 or 11.

13. A cell line according to Claim 12, being the CHO cell line CNCM I-1085.

14. A method for the preparation of the protein according to any one of Claims 1-3, 6 and 7, comprising transfecting eukaryotic host cells with an expression vector according to Claim 10 or 11, cultivating the transfected cells and obtaining said protein from the cultivated cells and/or the culture medium.

15. A method according to Claim 14 substantially as described herein in Example 1, 2 or 10.

16. Antibodies having binding specificity for the protein according to any one of Claims 1-3, 6 and 7.

17. Use of a protein according to any one of Claims 1-4, 6 and 7 or a conjugate of Claim 5, for the preparation of a medicament for treating, preventing or diagnosing diseases associated with over-production of SCF or of SCF-receptors.
